# EUROPÄISCHE PATENTSCHRIFT

(11) **EP 1 509 266 B1**
(45) Veröffentlichungstag und Bekanntmachung des Hinweises auf die Patenterteilung: **01.07.2009**
(21) Anmeldenummer: 03752661.3
(22) Anmeldetag: 23.04.2003
(51) Int. Cl.: A61M 11/00

(54) **SYSTEM UMFASSEND EINE DÜSE UND EIN HALTERUNGSSYSTEM**
SYSTEM COMPRISING A NOZZLE AND A FIXING SYSTEM
SYSTEME COMPRENANT UNE BUSE ET UN DISPOSITIF DE MAINTIEN

(30) Priorität: 16.05.2002 DE 10221732
(43) Veröffentlichungstag der Anmeldung: 02.03.2005
(73) Patentinhaber: Boehringer Ingelheim International GmbH, 55216 Ingelheim am Rhein (DE)
(72) Erfinder: GESER, Johannes, 55218 Ingelheim (DE); HOCHRAINER, Dieter, 57392 Oberkirchen (DE); WACHTEL, Herbert, 55411 Bingen (DE); DUNNE, Stephen, Great Finborough, Suffolk IP14 3AE (GB)
(86) Internationale Anmeldenummer: PCT/EP2003/004198
(87) Internationale Veröffentlichungsnummer: WO 2003/097139

(56) Entgegenhaltungen:
- WO-A-00/41814
- WO-A-94/07607
- WO-A-97/11784
- WO-A-97/12687
- US-A- 4 623 337
- US-A- 5 060 869
- US-A- 6 085 740
- US-E- R E30 486

## Beschreibung

Die Erfindung betrifft ein Düsensystem für eine Ausbringungsvorrichtung für Flüssigkeiten, wobei das Düsensystem eine Düse und eine die Düse in der Ausbringungsvorrichtung fixierenden Einrichtung beinhaltet. Die Ausbringungsvorrichtung, ein Zerstäuber, weist ein Flüssigkeitsreservoir auf, von welchem aus eine Flüssigkeit unter Druck durch die Düse gepresst wird. Die Düsenhalterung kann selbst von einer zweiten Halterung, z.B. in Form einer Überwurfmutter gehalten werden oder die Halterung selbst ist eine Überwurfmutter. Erfindungsgemäß weist die Halterung auf der Düsenauslassseite eine bestimmte Geometrie auf, die den Anteil von sich abscheidender ausgebrachter Flüssigkeit auf der Halterung minimiert.

Bevorzugt ist die vorliegende Erfindung Teil eines treibgasfreien Geräts zum Vernebeln von pharmazeutischen Flüssigkeiten. Ein solcher erfindungsgemäßer Vernebler dient beispielsweise zur Bereitstellung eines Aerosols aus Tröpfchen zur inhalativen Aufnahme durch den Mund- und Rachenbereich in die Lunge eines Patienten, zur nasalen Aufnahme oder zur Besprühung der Augenoberfläche.

### Stand der Technik

Die WO 91/14468 offenbart eine Vorrichtung zur treibgasfreien Verabreichung einer dosierten Menge eines flüssigen Arzneimittels zur inhalativen Anwendung. Eine Weiterentwicklung des Geräts wird in der WO 97/12687 ausführlich beschrieben. Auf die genannten Referenzen wird hiermit ausdrücklich Bezug genommen und die dort beschriebene Technologie wird im Rahmen der vorliegenden Erfindung als Respimat^{®}-Technologie bezeichnet. Unter diesem Begriff wird dabei insbesondere die Technologie verstanden, die einem Gerät gemäß den Figuren 6a und 6b der WO 97/12687 und der dazugehörigen Beschreibung zugrunde liegt. Mit solchen Geräten können treibgasfreie Flüssigkeiten einfach zerstäubt werden.

In einem solchen Inhalator werden flüssige Arzneistoffformulierungen in einem Reservoir gelagert. Von dort aus werden sie über ein Steigrohr in eine Druckkammer befördert, um dann weiter durch eine Düse gepresst zu werden.

Die Düse wird von einem Düsenhalter gehalten und dieser von einer Überwurfmutter. Die Düse weist eine Flüssigkeitseinlassseite und eine Flüssigkeitsauslassseite auf. Auf der Flüssigkeitseinlassseite befindet sich eine Öffnung durch die eine von der Druckkammer her kommende Flüssigkeit in die Düse eintreten kann. Auf der gegenüberliegenden Seite, der Stirnseite der Düse, tritt die Flüssigkeit dann durch zwei Düsenöffnungen aus, die so ausgerichtet sind, dass die aus den Öffnungen austretenden Flüssigkeitsstrahlen aufeinanderprallen und dadurch zerstäubt werden. Die Düsenöffnungen sind so im Inhalator angebracht, dass sie mit der Außenumgebung in unmittelbarem Kontakt stehen.
Dies wird erreicht, indem der gesamte Bereich des Düsenhalters und der Überwurfmutter, welcher über den Düsenöffnungen liegt, eine durchgehende Ausnehmung (auch Loch oder Bohrung) aufweist, die den Weg für einen aus der Düse austretenden Flüssigkeitsstrahl oder ein austretendes Aerosol frei gibt, um über das Mundstück den Zerstäuber zu verlassen.

Im Bereich des Düsenhalters ist diese Ausnehmung trichterförmig, im Bereich der Überwurfmutter ist diese Ausnehmung von der Gestalt eines gleichförmigen Zylinders. Der Übergang zwischen Düsenhalter und Überwurfmutter weist dabei eine scharfe Kante auf, so dass der Querschnitt der Ausnehmung wie ein L wirkt, dessen querliegender Balken leicht nach unten geneigt ist. Die gesamte Ausnehmung vor der Düsenöffnung, die sich aus der Ausnehmung des Düsenhalters und der Ausnehmung der Überwurfmutter zusammensetzt weist im Kniebereich dieses L eine Unstetigkeitsstelle auf: Die Ausnehmung erweitert sich dis-kontinuierlich, d.h. vom Fußpunkt aus betrachtet erweitert sie sich zunächst, um dann im Bereich des Übergangs von dem Düsenhalter zur Überwurfmutter scharf vertikal abzuknicken. Die vertikale Richtung entspricht der Sprührichtung der austretenden Flüssigkeit, also der auf der Düsenaußenseite (Stirnseite) Senkrecht stehenden.

Diese Inhalatoren bringen zumeist Formulierungen auf Basis von Wasser oder Wasser-Ethanol-Gemischen aus. Dabei können sie eine kleine Menge einer flüssigen Formulierung in der therapeutisch notwendigen Dosierung binnen weniger Sekunden in ein therapeutisch-inhalativ geeignetes Aerosol vernebeln. Mit dem Gerät können Mengen von weniger als 100 Mikroliter mit beispielsweise einem Hub zu einem Aerosol mit einer durchschnittlichen Teilchengröße von weniger als 20 Mikrometern so vernebelt werden, daß der inhalierbare Anteil des Aerosols bereits der therapeutisch wirksamen Menge entspricht.
In diesen Vernebler mit Respimat^{®} -Technologie wird eine Arzneimittellösung mittels eines hohen Drucks von bis zu 500 bar in eine lungengängige Aerosolwolke niedriger Geschwindigkeit überführt, die dann vom Patienten eingeatmet werden kann.

Dabei kann sich ein geringer Teil der Flüssigkeit als Film oder als Ansammlung kleiner Tröpfchen von außen auf die Stirnseite der Düse oder die Stirnseite des Halterungssystems der Düse oder an der Innenseite des Mundstückes niederschlagen. Dieser Anteil der Flüssigkeit wird im Rahmen dieser Erfindungsbeschreibung auch als Mundstückanteil bezeichnet. Diese Mundstückanteil reduziert die ausgebrachte Menge an Flüssigkeit, so dass der inhalierbare Anteil der ausgebrachten Menge um den Mundstückanteil reduziert ist.

Der Anteil an niedergeschlagener Flüssigkeit muss nicht bei jedem Hub konstant sein, sondern kann von mehreren Faktoren wie der Orientierung des Geräts im Raum während der Aerosolisierung oder der Umgebungstemperatur, Luftfeuchte etc. abhängen. Dies führt zum einen zu einer gewissen, wenn auch nur geringen Variabilität der ausgebrachten Menge, die dann für den Patienten zur Aufnahme zur Verfügung steht (delivered dose). Von der ausgebrachten Menge (delivered dose) weist ein Teil eine so kleine Tröpfchengröße auf, dass die Tröpfchen tief in die Lunge eingeatmet werden können, dieser Anteil heißt inhalierbarer Anteil. Im Rahmen der vorliegenden Erfindungsbeschreibung wird jedoch nicht ausdrücklich zwischen inhalierbarem Anteil und der Gesamtmenge an Aerosol unterschieden, die dem Patienten zum Einatmen zur Verfügung steht, wenn nicht anderweitig gekennzeichnet oder aus dem Zusammenhang klar entnehmbar.

Die niedergeschlagene Flüssigkeit kann auch zu einer Verunreinigung der Außenoberfläche des Düsensystems oder des Mundstückes führen, was seinerseits die pharmazeutische Qualität einer nächsten Aerosolwolke beeinflussen kann.

Wenngleich die beiden Effekte gerade bei Geräten der Respimat^{®}-Technologie gering sind, ist es aus Gründen der Qualitätssicherung ein Anliegen, solche Effekte zu minimieren.

Es wurde nun gefunden, dass sich in solchen Vorrichtungen zum Ausbringen von Flüssigkeiten der Anteil der an der Außenseite des Düsensystems niedergeschlagenen Flüssigkeitsmenge durch eine bestimmte Geometrie der Düse bzw. Düsenhalterung verringern lässt. Es hat sich nämlich überraschend gezeigt, dass der Mundstückanteil reduziert werden kann, wenn der gesamte Bereich oberhalb der Düsenöffnung (d.h. der Bereich, den die ausgebrachte Flüssigkeit auf dem Weg zum Mundstück "durchfliegt") trichterförmig ist und keine Kante aufweist.

### Beschreibung der Erfindung

Es ist eine Aufgabe der Erfindung die Variabilität des durch eine Vorrichtungen zum Ausbringen von pharmazeutischen Flüssigkeiten, wie Zerstäubern, Inhalatoren etc., ausgebrachten Anteils der Flüssigkeit zu reduzieren.

Es ist eine weitere Aufgabe der vorliegenden Erfindung den Anteil von Flüssigkeit, der sich aus einer erzeugten Aerosolwolke an der Vorrichtung zum Ausbringen der pharmazeutischen Flüssigkeit niederschlägt, zu verringern.

Es ist damit eine weitere Aufgabe der Erfindung den inhalierbaren Anteil der ausgebrachten Menge zu erhöhen und den Mundstückanteil zu reduzieren.

Eine weitere Aufgabe besteht darin, die Qualität mit der Zerstäuber der Respima^{®}-Technologie eine Flüssigkeit ausbringen, zu optimieren.

### Beschreibung der Erfindung im Detail

Gelöst wird diese Aufgabe durch ein Düsensystem nach Anspruch 1.

Vorteilhaft sind dabei Ausführungsformen des Düsensystems, bei dem die Ausnehmung von trichterförmiger Gestalt, vorzugsweise von konusförmiger Gestalt ist.

Unter dem Begriff einer "sich kontinuierlicher erweiternden Ausnehmung" wird dabei im Rahmen der vorliegenden Erfindung eine Fläche verstanden, deren Rand im Querschnitt eine stetigen Verlauf aufweist. Diese Betrachtungen beziehen sich auf den makroskopisch sichtbaren Bereich. Unter "stetigem Verlauf" wird verstanden, dass keine Sprünge von mehr als 0,5 mm, bevorzugt mehr als 0,1mm auftreten.

Im Querschnitt stellt sich der Rand dieser "sich kontinuierlicher erweiternden Ausnehmung" bevorzugt als gerade, elliptische, hyperbolische, konvexe oder konkave Linie auf. In jedem Fall weist der Rand einen stetigen Verlauf auf. Die Ausnehmung erweitert sich ebenfalls stetig und geht nicht in einen zylinderförmigen Bereich über.

Der Bereich der Ausnehmung mit dem engsten Durchmesser, der Fußpunkt, liegt auf der Seite des Düsenhalters, der an die Stirnseite der Düse grenzt.

Der Bereich der Ausnehmung mit dem größten Durchmesser, der Scheitelpunkt, liegt auf der gegenüber liegenden Seite, also der zur Stirnseite der Düse parallel liegenden Außenseite des Düsenhalters oder im Fall einer Überwurfmutter deren dazu parallel liegenden Außenseite.

Der kleiner Durchmesser der Ausnehmung liegt zwischen 0,1 mm und 2 mm, bevorzugt zwischen 0,6 mm und 1,0 mm.

Der größere Durchmesser der Ausnehmung liegt zwischen 3 mm und 10 mm, bevorzugt zwischen 5 mm und 8 mm.

Der Übergang des Fußendes der Ausnehmung zur Stirnseite der Düse kann dabei als Kante ausgebildet sein oder stetig sein, wie oben definiert, also kantenlos.

Für das erfindungsgemäße System kann gegebenenfalls auf eine Überwurfmutter verzichtet werden, wenn der Düsenhalter selbst diese Aufgabe übernimmt.

Bevorzugt wiest das Düsensystem eine Überwurfmutter auf. In diesem Fall ist der Übergang zwischen Düsenhalter und Überwurfmutter kantenlos ausgebildet, d.h. der stetige Verlauf der Ausnehmung wird nicht unterbrochen. Bevorzugt findet in diesem Bereich keine Änderung der Steigung der sich bevorzugt konisch erweiternden Ausnehmung statt.

Zur Lösung der gestellten Aufgabe kann zudem durch Veränderung der Abstände der Düsenöffnung und der Neigungswinkel, mit denen Flüssigkeitsstrahlen aus den Düsenöffnungen ausgebracht werden, beigetragen werden.

Als nächstliegender Stand der Technik wird Dokument USRE30486 E angesehen. Dieses Dokument offenbart alle Merkmale des Oberbergriffs des Anspruch 1.

Der vorliegenden Erfindung liegen dabei Düsensysteme zugrunde wie sie beispielsweise in der EP 0664733 oder der EP 1017469 beschrieben sind. Bevorzugt handelt es sich dabei um Düsen aus wenigstens zwei aufeinanderliegenden Platten, wovon wenigstens eine der Platten eine zweite Mikrostruktur aufweist, so dass die übereinander liegenden Platten auf einer Seite einen Flüssigkeitseinlass definieren, dem sich ein Kanalsystem und/oder ein Filtersystem anschließt, welches dann in den Flüssigkeitsauslässen mündet.

Derartig mikrostrukturierte Düsenkörper sind beispielsweise in der WO 94/07607 oder der WO 99/16530 beschrieben. Eine weitere Ausführungsform offenbart die deutsche Patentanmeldung mit der Anmeldenummer 10216101.1. Auf alle Dokumente wird hiermit ausdrücklich Bezug genommen.
Bezüglich der WO 94/07607 wird insbesondere auf Figur 1 und deren Beschreibung verwiesen. Der Düsenkörper besteht z.B. aus zwei fest miteinander verbundenen Platten aus Glas und/oder Silizium, von denen wenigstens eine Platte einen oder mehrere mikrostrukturierte Kanäle aufweist, welche die Düseneinlaßseite mit der Düsenauslaßseite verbinden. Auf der Düsenauslaßseite kann mindestens eine - erfindungsgemäß bevorzugt sind zwei - runde oder nicht-runde Öffnung(en) von 2 bis 10 Mikrometer Tiefe und 5 bis 15 Mikrometern Breite sein, wobei die Tiefe bevorzugt bei 4, 5 bis 6,5 Mikrometer und die Länge bei 7 bis 9 Mikrometer liegt.

Auf dem Basisteil kann auf der ebenen Oberfläche ein Satz von Kanälen ausgebildet sein, um im Zusammenwirken mit der im wesentlichen ebenen Oberfläche des Deckenteils eine Vielzahl von Filterdurchgangswegen zu schaffen (Filterkanäle). Daneben kann das Basisteil eine Plenumkammer aufweisen, deren Decke wiederum durch das Deckenteil gebildet wird. Diese Plenumkammer kann den Filterkanälen vor- oder nachgeschaltet sein. Es können auch zwei derartige Plenumkammern ausgebildet sein. Ein anderer Satz von Kanälen auf der im wesentlichen ebenen Oberfläche des Basisteils, der den Filterkanälen nachgeschaltet ist, bildet zusammen mit dem Deckenteil einen Satz von Kanälen, die eine Vielzahl von Düsenauslaßdurchgangswegen schaffen.
Bevorzugt liegt der Gesamtquerschnittsflächenbereich der Düsenauslässe bei 25 bis 500 Quadratmikrometern. Der gesamte Querschnittsflächenbereich beträgt bevorzugt 30 bis 200 Quadratmikrometer.
In einer anderen Ausführungsform weist auch diese Düsenkonstruktion nur eine einzige Düsenöffnung auf.
In anderen Ausführungsformen dieser Art fehlen die Filterkanäle und/oder die Plenumkammer.

Bevorzugt werden die Filterkanäle durch Vorsprünge gebildet, die zick-zackförmig angeordnet sind. So bilden beispielsweise mindestens zwei Reihen der Vorsprünge eine solche zick-zack-Konfiguration. Auch können mehrere Reihen von Vorsprüngen ausgebildet sein, wobei die Vorsprünge jeweils seitlich zueinander versetzt sind, um dadurch zu diesen Reihen windschiefe weitere Reihen aufzubauen, wobei dann diese zuletzt beschriebenen Reihen die Zick-Zack-Konfiguration bilden. In solchen Ausführungsformen kann der Einlass und der Auslass jeweils einen Längsschlitz für unfiltriertes bzw. filtriertes Fluid aufweisen, wobei jeder der Schlitze im wesentlichen genauso breit ist wie der Filter und im wesentlichen genauso hoch ist wie die Vorsprünge auf den Einlass- bzw. Auslassseiten des Filters. Der Querschnitt der durch die Vorsprünge gebildeten Durchgangspassagen kann jeweils senkrecht zur Strömungsrichtung des Fluids stehen und kann - betrachtet in Strömungsrichtung - von Reihe zu Reihe abnehmen. Auch können die Vorsprünge, die näher zur Einlassseite des Filters angeordnet sind, größer sein als die Vorsprünge, die näher an der Auslassseite des Filters angeordnet sind. Daneben kann sich auch der Abstand zwischen dem Basisteil und dem Deckteil in dem Bereich von der Düseneinlaßseite zur Düsenauslaßseite verjüngen.
Die Zick-Zack-Konfiguration, die von den wenigstens zwei Reihen von Vorsprüngen gebildet wird, weist einen Neigungswinkel alpha von bevorzugt 20° bis 250° auf.

Weitere Einzelheiten dieser Düsenkonstruktion können der WO-94/07607 entnommen werden. Auf diese Schrift wird hiermit inhaltlich Bezug genommen, insbesondere auf Figur 1 und deren Beschreibung.

Bei Ausführungsformen der Düse mit mehreren Düsenöffnungen sind bevorzugt alle auf einer gemeinsamen Seite ausgebildet. In solchen Fällen können die Düsenöffnungen so orientiert sein, dass die daraus austretenden Flüssigkeitsstrahlen vor der Düsenöffnung aufeinanderprallen. Für solche Systeme werden Düsen mit wenigstens zwei Öffnungen benötigt. Derartige Düsen sind erfindungsgemäß bevorzugt.

Die Düse kann in einer elastomeren Manschette eingebettet sein, wie sie in der WO 97/12683 beschreiben wird. Eine solche Manschette ist in der einfachsten Form ein Ring oder Körper mit einer Öffnung, in die die Düse eingesetzt werden kann. Diese Öffnung umfasst den Düsenblock über seiner gesamten Mantelfläche, d.h. die Fläche die senkrecht zu der bevorzugt linearen Achse steht, die durch die Düseneinlassseite und die Düsenauslaßseite gebildet wird. Die Manschette ist nach oben und unten offen, um weiter die Flüssigkeitszufuhr zur Düseneinlassseite der Düse, noch die Ausbringung der Flüssigkeit zu behindern. Diese Manschette kann wiederum in eine zweite Manschette eingesetzt werden. Die äußere Gestalt der ersten Manschette ist bevorzugt kegelförmig. Entsprechend ist die Öffnung der zweiten Manschette geformt. Die erste Manschette kann aus einem Elastomer bestehen.

Die Düse wird von der erfindungsgemäßen Einrichtung zum Halten gehalten.

Eine solche Düse - ggf. inklusive der Manschette - ist dabei Teil eines Düsensystems, durch das die Düse an einem definierten Ort - bevorzugt von außen in Richtung des Hohlkolbens - in der Ausbringungsvorrichtung gehalten wird. Erfindungsgemäß besteht ein solches Düsensystem demnach aus einer Düse und einem Düsenhalter und gegebenenfalls einer Überwurfmutter. Dabei weisen alle Elemente jeweils eine Stirnseite auf. Diese ist die Seite, die von der Seite der Düse mit der Düsenöffnung weg orientiert ist, also nach außen weist. Die Innenseite der Stirnseite des Düsenhalters oder der Überwurfmutter kann die Flüssigkeitsauslassseite der Düse berühren und dadurch die zum Halten der Düse notwendige Kraft in Richtung der Flüssigkeitseinlassseite der Düse ausüben. Die Stirnseite des Düsenhalters und/oder der Überwurfmutter weisen eine durchgehende Bohrung oder Loch in Form einer Ausnehmung auf, durch die das Aerosol aus der Düse austreten kann. Daher liegen die Düsenöffnungen in oder in direkter Linie unterhalb der Bohrung, bzw. der Ausnehmung

Die Ausnehmung ist bevorzugt als eine von den Düsenöffnungen sich kontinuierlich erweiternde innere Ausnehmung ausgebildet. Vorteilhaft sind dabei Ausführungsformen des Düsensystems, bei dem die Ausnehmung von trichterförmiger Gestalt, vorzugsweise von konusförmiger Gestalt ist.
Bei Düsen mit wenigstens zwei Düsenöffnungen, die so orientiert sind, dass die zwei Flüssigkeitsstrahlen, die den Düsenkörper verlassen, sich treffen, befindet sich der Impaktionspunkt, dem Punkt an dem sich die Flüssigkeitsstrahlen treffen und zu einem Aerosol zerstäubt werden, bevorzugt in der Nähe des Fußes der Ausnehmung, also in der Nähe der Düsenöffnung. Es ist offensichtlich, dass in einem solchen Fall die Ausnehmung zu den besonders für das Niederschlagen von Flüssigkeit gefährdeten Bereichen gehört.

Diese Erfindung wird bevorzugt in einem Vernebler der Respimat^{®}-Technologie eingesetzt, der im Folgenden näher beschrieben wird.

Im wesentlichen besitzt der bevorzugte Zerstäuber ein unteres und ein oberes gegeneinander drehbar gelagertes Gehäuse, wobei in dem Gehäuseoberteil ein Federgehäuse mit Springfeder ausgebildet ist, welche durch Drehen der beiden Gehäuseteile über ein Sperrspannwerk bevorzugt in Form eines Schraubgewindes oder Getriebes gespannt und durch Drücken eines Auslöseknopfs am Gehäuseoberteil entspannt wird. Dadurch wird ein Abtriebsflansch bewegt, der mit einem Hohlkolben verbunden ist, an dessen unterem Ende ein Behälter aufgesteckt werden kann und an dessen oberem Ende sich ein Ventil und eine Druckkammer befindet, die mit der Düse oder dem Düsensystem, die (das) im nach oben hin offen Bereich des Gehäuseoberteils ausgebildet ist, in einer eine Flüssigkeit leitenden Verbindung steht. Die Flüssigkeit wird vom Hohlkolben angesaugt und zur Druckkammer gepumpt, von wo sie aus durch die Düse als Aerosol ausgebracht wird.

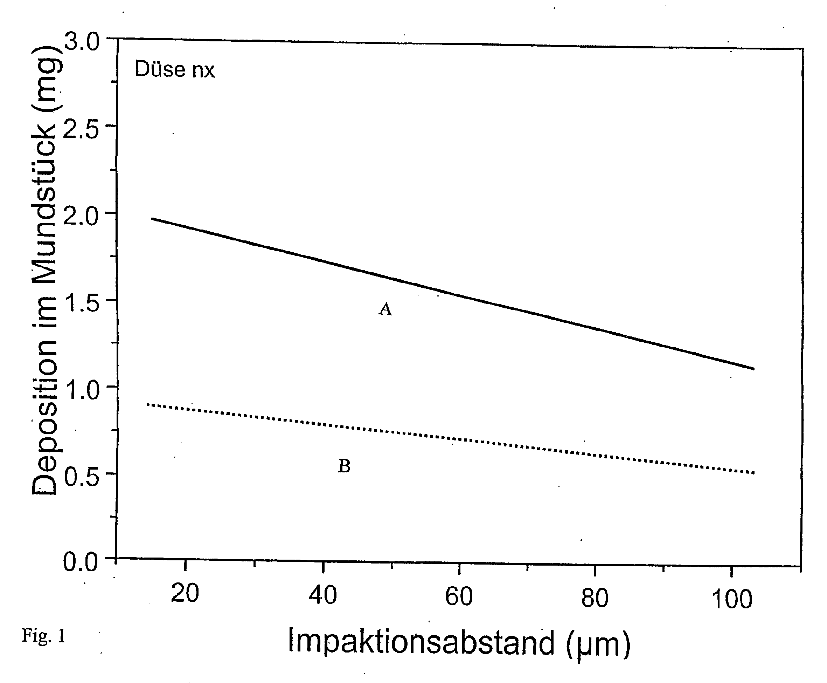

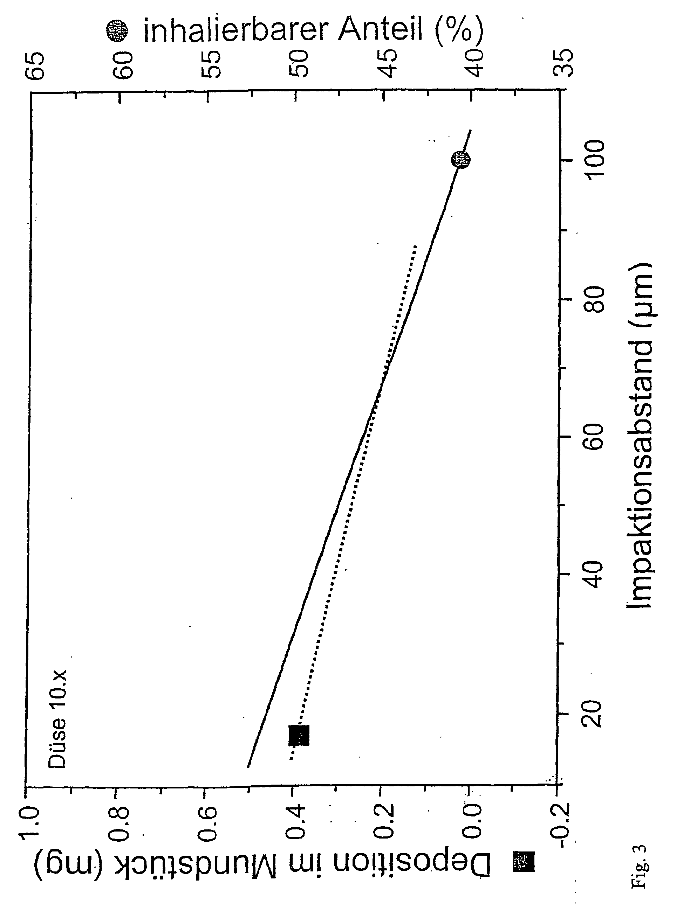

Der Hohlkolben mit Ventilkörper entspricht einer in der WO 97/12687 offenbarten Vorrichtung. Er ragt teilweise in den Zylinder des Pumpengehäuses hinein und ist im Zylinder axial verschiebbar angeordnet. Insbesondere wird auf die Figuren 1-4 - insbesondere Figur 3 - und die dazugehörigen Beschreibungsteile Bezug genommen. Der Hohlkolben mit Ventilkörper übt auf seiner Hochdruckseite zum Zeitpunkt des Auslösens der Feder einen Druck von 5 bis 60 MPa (etwa 50 bis 600 bar), bevorzugt 10 bis 60 MPa (etwa 100 bis 600 bar) auf das Fluid, die abgemessene Wirkstofflösung aus.
Der Ventilkörper ist bevorzugt an dem Ende des Hohlkolbens angebracht, das dem Düsenkörper zugewandt ist. Der Ventilkörper steht flüssigkeitsleitend mit der Düse in Verbindung.
Ferner weist die Ausbringungsvorrichtung ein Sperrspannwerk auf. Dieses enthält eine Feder, bevorzugt eine zylindrische schraubenförmige Druckfeder, als Speicher für die mechanische Energie. Die Feder wirkt auf den Abtriebsflansch, einemSprungstück, dessen Bewegung durch die Position eines Sperrglieds bestimmt wird. Der Weg des Abtriebsflansches wird durch einen oberen und einen unteren Anschlag präzise begrenzt. Die Feder wird bevorzugt über ein kraftübersetzendes Getriebe, z.B. ein Schraubschubgetriebe, durch ein äußeres Drehmoment gespannt, das beim Drehen des Gehäuseoberteils gegen das Federgehäuse im Gehäuseunterteil erzeugt wird. In diesem Fall enthalten das Gehäuseoberteil und der Abtriebsflansch ein ein- oder mehrgängiges Keilgetriebe.

Das Sperrglied mit einrückenden Sperrflächen ist ringförmig um den Abtriebsflansch angeordnet. Es besteht z.B. aus einem in sich radial elastisch verformbaren Ring aus Kunststoff oder aus Metall. Der Ring ist in einer Ebene senkrecht zur Zerstäuberachse angeordnet. Nach dem Spannen der Feder schieben sich die Sperrflächen des Sperrgliedes in den Weg des Abtriebsflansches und verhindern das Entspannen der Feder. Das Sperrglied wird mittels einer Taste ausgelöst. Die Auslösetaste ist mit dem Sperrglied verbunden oder gekoppelt. Zum Auslösen des Sperrspannwerkes wird die Auslösetaste parallel zur Ringebene, und zwar bevorzugt in den Zerstäuber hinein, verschoben; dabei wird der verformbare Ring in der Ringebene verformt. Konstruktive Details des Sperrspannwerkes sind in der WO 97/20590 beschrieben.

Das Gehäuseunterteil wird in axialer Richtung über das Federgehäuse geschoben und verdeckt die Lagerung, den Antrieb der Spindel und den Vorratsbehälter für das Fluid.

Beim Betätigen des Zerstäubers wird das Gehäuseoberteil gegen das Gehäuseunterteil gedreht, wobei das Gehäuseunterteil das Federgehäuse mitnimmt. Dabei wird die Feder über das Schraubschubgetriebe zusammengedrückt und gespannt, und das Sperrwerk rastet selbsttätig ein. Der Drehwinkel ist bevorzugt ein ganzzahliger Bruchteil von 360 Grad, z.B. 180 Grad. Gleichzeitig mit dem Spannen der Feder wird das Abtriebsteil im Gehäuseoberteil um einen vorgegebenen Weg verschoben, der Hohlkolben wird innerhalb des Zylinders im Pumpengehäuse zurückgezogen, wodurch eine Teilmenge des Fluids aus dem Vorratsbehälter in den Hochdruckraum vor der Düse eingesaugt wird.

In den Zerstäuber können gegebenenfalls nacheinander mehrere, das zu zerstäubende Fluid enthaltende, austauschbare Vorratsbehälter eingeschoben und benutzt werden. Der Vorratsbehälter enthält die treibgasfreie Aerosolzubereitung.

Der Zerstäubungsvorgang wird durch leichtes Eindrücken der Auslösetaste eingeleitet. Dabei gibt das Sperrwerk den Weg für das Abtriebsteil frei. Die gespannte Feder schiebt den Hohlkolben in den Zylinder des Pumpengehäuses hinein. Das Fluid tritt aus der Düse des Zerstäubers in zerstäubter Form aus. Die flüssige Arzneimittelzubereitung trifft mit einem Eingangsdruck von bis zu 600 bar, bevorzugt 200 bis 300 bar auf den Düsenkörper und wird über die Düsenöffnungen in ein inhalierbares Aerosol zerstäubt. Die bevorzugten Teilchengrößen des Aerosols liegen bei bis zu 20 Mikrometern, bevorzugt 3 bis 10 Mikrometern. Dabei werden Volumina von 10 bis 50 Mikroliter bevorzugt ausgebracht, besonders bevorzugt sind Volumina von 10 bis 20 Mikroliter, ganz besonders bevorzugt ist ein Volumen von 15 Mikroliter pro Hub.

Die Bauteile des Zerstäubers (Verneblers) sind aus einem der Funktion entsprechend geeigneten Material. Das Gehäuse des Zerstäubers und - so weit es die Funktion erlaubt - auch andere Teile sind bevorzugt aus Kunststoff, z.B. im Spritzgießverfahren, hergestellt. Für medizinische Zwecke werden physiologisch unbedenkliche Materialien verwendet.

Bevorzugt ist ein erfindungsgemäßer Vernebler von zylinderähnlicher Form und weist eine handliche Größe von weniger als 9 bis 15 cm in der Länge und 2 bis 4 cm in der Breite auf, so dass sie vom Patienten jederzeit mitgeführt werden kann.

Weitere konstruktive Details sind in den PCT-Anmeldungen WO 97/12683 und WO 97/20590 offenbart, auf die hiermit inhaltlich Bezug genommen wird.

Im folgenden wird die Erfindung anhand von Figuren näher erläutert.

### Figuren

Fig. 1 Grafik zur Untersuchung von Düsensystemen mit zwei gegeneinander ausgerichteten Düsenöffnungen: Abhängigkeit des Mundstückanteils ("Deposition im Mundstück") von der Impaktionshöhe h für ein Düsensystem mit sich dis-kontinuierlich ausdehnender Ausnehmung und für ein erfindungsgemäßes Düsensystem mit konischer Ausnehmung,
Fig. 2 Grafik zur Untersuchung von Düsensystemen mit zwei gegeneinander ausgerichteten Düsenöffnungen: Abhängigkeit der Aerosolqualität von der Impaktionshöhe,
Fig. 3 Grafik zur Untersuchung von Düsensystemen mit zwei gegeneinander ausgerichteten Düsenöffnungen: Abhängigkeiten des Mundstückanteils und der Qualität des inhalierbaren Anteils von der Impaktionshöhe,
Fig. 4 Grafik zur Untersuchung von Düsensystemen mit zwei gegeneinander ausgerichteten Düsenöffnungen: Abhängigkeit des Mundstückanteils vom Konuswinkel 2θ bei Düsenhalterungssystemen mit konusförmiger Ausnehmung,
Fig. 5, 6 Düsen mit zwei gegeneinander ausgerichteten Düsenöffnungen: Einfluss des Impaktionswinkels α auf den inhalierbaren Anteil und den Mundstückteil bei Düsenhalterungssystemen mit konusförmiger Ausnehmung,
Fig.7 Darstellung einer ersten Ausführungsform eines Düsensystems in der Seitenansicht, teilweise geschnitten,
Fig.8 Darstellung einer zweiten Ausführungsform eines Düsensystems in der Seitenansicht, teilweise geschnitten,
Fig.9 schematische Darstellung eines erfindungsgemäßen Düsensystems in der Seitenansicht, geschnitten,
Fig.10 schematische Darstellung einer Ausführungsform eines Düsenkörpers in der Seitenansicht, geschnitten.
Fig.11a/b Schema des Verneblers vom Respimat^{®} -Typ.

Figur 1 zeigt die Abhängigkeit des Mundstückanteils ("Deposition im Mundstück") von der Impaktionshöhe h für ein Düsensystem mit sich dis-kontinuierlich ausdehnender Ausnehmung (A) und für ein erfindungsgemäßes Düsensystem mit konischer Ausnehmung (B). Diese Darstellung zeigt die Abhängigkeit des Mundstückanteils von der Impaktionshöhe. Demanch kann eine Reduzierung des Mundstückanteils durch Vergrößerung der Impaktionshöhe h erzielt werden.
Andererseits läßt sich Figur 1 auch entnehmen, daß die erfindungsgemäße, spezielle Ausgestaltung des Düsensystems im Bereich vor den Düsenöffnungen zu einer erheblichen Reduzierung des Mundstückanteils im Vergleich zu herkömmlichen Systemen führt. So kann beispielsweise der Mundstückanteil bei einer Impaktionshöhe h = 25 µm von etwa 1,9 mg auf 0,8 mg verkleinert werden, was einer Reduzierung von ca. 60 % entspricht.

Die Reduzierung des Mundstückanteils hat dabei zwei positive Effekte. Zum einen wird mit einer Minimierung der Mundstückanteilsmasse die ausgebrachte Menge maximiert, wodurch auch in günstiger Weise auf den inhalierbaren Anteil Einfluß genommen wird, der hierdurch grundsätzlich größer wird. Damit wird entscheidend zur Lösung einer der Erfindung zugrunde liegende Teilaufgabe, nämlich den inhalierbaren Anteil zu maximieren, beigetragen.

Zum anderen wird durch die Reduzierung des Mundstückanteils der Einfluß einer Variabilität des Mundstückanteils gemindert. Infolge der geringen Mundstückanteilsmasse haben Schwankungen der Mundstückanteilsmasse nur noch geringe Schwankungen der ausgebrachten Masse und damit des inhalierbaren Anteils zur Folge. Der inhalierbare Anteil weist nunmehr eine hohe Reproduzierbarkeit d.h. eine geringe Variabilität auf. Die Problematik des nicht konstanten, Toleranzen unterworfenen Mundstückanteils hat nur noch marginale Bedeutung. Damit wird auch die der Erfindung zugrunde liegende zweite Teilaufgabe, eine hohe Reproduzierbarkeit d.h. eine geringe Variabilität des inhalierbaren Anteils zu gewährleisten, gelöst.

Entscheidend für den Erfolg des erfindungsgemäßen Düsensystems ist aber auch, daß durch ein und die gleiche Maßnahme nicht nur der Mundstückanteil minimiert sondern gleichzeitig der inhalierbare Anteil maximiert wird.

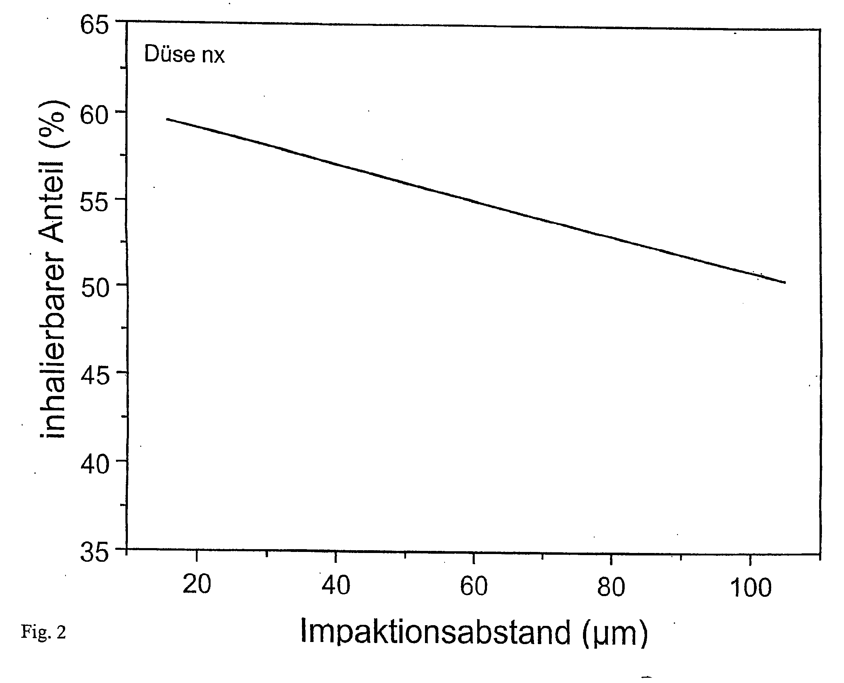

Erfindungsgemäß zeigt sich, dass es bei der Reduktion des Mundstückanteils bei Düsensystemen mit Düsen mit zwei Düsenöffnungen, die so ausgerichtet sind, dass sich daraus austretende Strahlen in einem Punkt vor der Düse treffen (Impaktionspunkt), nicht zielführend ist, allein die Impaktionshöhe h zu erhöhen (Figur 1). Dies liegt daran, daß die beiden Strahlen sich auch tatsächlich treffen müssen, was eine möglichst kleine Impaktionshöhe h verlangt. Des Weiteren sollen die Strahlen gebündelt aufeinander treffen, bevor sie in Tröpfchen auseinander fallen. Außerdem hat sich überraschend gezeigt, dass die Größe der Impaktionshöhe h auch Einfluß auf die Zerstäubungsgüte und damit auf den inhalierbaren Anteil in der Art hat, daß mit zunehmender Impaktionshöhe h die Zerstäubungsgüte bzw. der inhalierbare Anteil abnimmt. Es liegen dann mit zunehmender Impaktionshöhe h mehr größere und weniger kleine Partikel vor. Diesen Einfluß zeigt Figur 2, wobei der inhalierbare Anteil als der Teil angesehen wird, der Teilchen mit einem Durchmesser kleiner 5,8µm aufweist. Auch hier zeigt sich der vorteilhafte Effekt des erfindungsgemäßen Düsensystems gegenüber einem Düsensystem mit sich dis-kontinuierlich ausdehnender Ausnehmung.

In Figur 3 ist der Mundstückanteil in Milligramm und der inhalierbare Anteil in Volumenprozent (Volumenanteil des von einem Laserstrahls erfaßten Aerosols, welches Teilchen mit Durchmessern kleiner 5,8µm enthält) in Abhängigkeit von der Impaktionshöhe h dargestellt. Beispielsweise nimmt für einen Impaktionswinkel α=75° der Mundstückanteil mit zunehmender Impaktionshöhe h rapide ab. Gleichzeitig nimmt aber der inhalierbare Anteil d.h. die Zerstäubungsgüte nicht in gleichem Maße ab.

Berücksichtigt man weiter, daß nicht nur die Zertäubungsgüte - gekennzeichnet durch den inhalierbaren Anteil in Volumenprozent - positiv beeinflußt wird, sondern ebenfalls durch Reduzierung der absoluten Mundstückanteilsmasse die tatsächlich ausgebrachte Masse gesteigert wird, wird ersichtlich, daß der absolute inhalierbare Anteil wesentlich vergrößert werden kann.

Erfindungsgemäß wurde gefunden, dass Ausführungsformen des Düsensystems vorteilhaft sind, wenn die Ausnehmungen vor der Düsenöffnung von konusförmiger Gestalt ist und diese einen Konuswinkel 2θ aufweist, der im Bereich zwischen 55° und 155°, vorzugsweise im Bereich zwischen 70° und 140° liegt. Günstig sind insbesondere Düsensysteme, bei denen die Ausnehmung von konusförmiger Gestalt einen Konuswinkel 2θ aufweist, der im Bereich zwischen 70° bis 85° oder im Bereich zwischen 95° und 140°, insbesondere im Bereich zwischen 105° und 125° liegt.

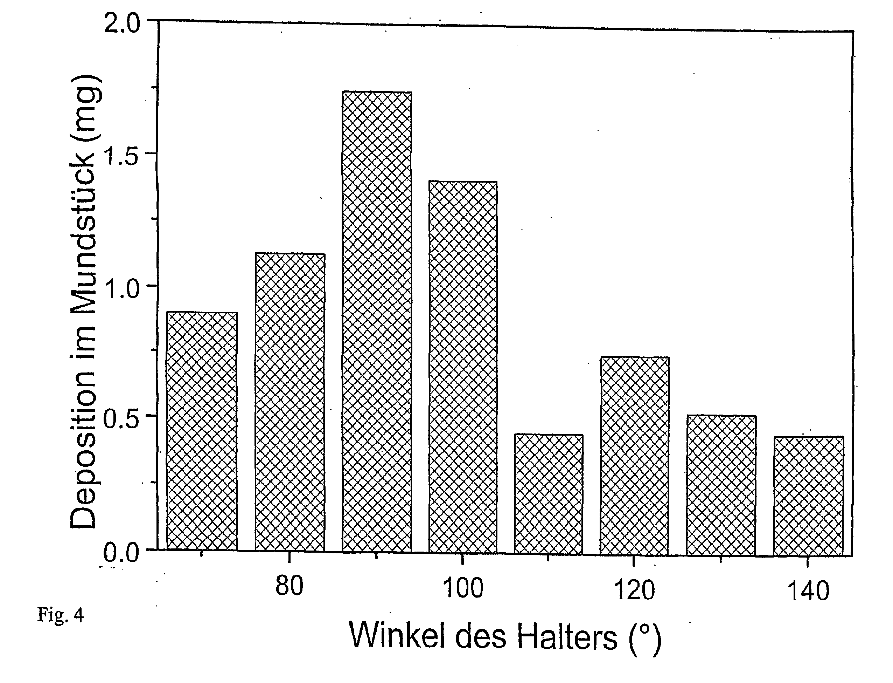

Die Vorteile dieser Ausführungsformen ergeben sich aus Figur 4, in welcher in Form eines Balken-Diagramms der Mundstückanteil in Milligramm für verschiedene Konuswinkel 2θ aufgetragen ist. Sämtliche Ausführungsformen verfügen über eine im Vergleich zum Stand der Technik geringe Mundstückanteilsmasse von maximal 1,75mg (Konuswinkel 2θ=90°). Noch geringere Mundstückteilsmassen weisen die Ausführungsformen auf, welche Konuswinkel 2θ im Bereich zwischen 70° bis 85° oder im Bereich zwischen 95° und 140°, insbesondere im Bereich zwischen 105° und 125° aufweisen. Ein Minimum wird bei einem Konuswinkel 2θ=110° erzielt.

Einweitere Aspekt der vorliegenden Erfindung betrifft bestimmte Düsen, die vorteilhaft in die erfindungsgemäßen Düsensysteme eingebaut werden können. Diese Düsen sind dadurch gekennzeichnet, daß der Kollisionspunkt, in dem sich die Strahlen treffen, eine Impaktionshöhe h über den Düsenöffnungen aufweist, die im Bereich zwischen 20µm und 85µm, vorzugsweise im Bereich zwischen 25µm und 75 µm liegt. Liegt die Impaktionshöhe im angegebenen Bereich kann den verschiedenen zu berücksichtigenden Zielsetzungen insgesamt in vorteilhafter Weise Rechnung getragen werden, wobei insbesondere eine geringer Mundstückanteil und ein sicheres Aufeinanderlenken der Flüssigkeitsstrahlen bei einem hohen inhalierbaren Anteil realisiert wird.

Günstig sind Düsen, bei denen der Kollisionspunkt, in dem sich die Strahlen treffen, eine Impaktionshöhe h über den Düsenöffnungen aufweist, die im Bereich zwischen 35 µm und 75 µm liegt. In diesem Bereich der Impaktionshöhe wird eine Optimierung der sich gegenseitig beeinflußenden Parameter erreicht.

Vorteilhaft sind Ausführungformen der Düsen, bei denen der Winkel α in einem Bereich zwischen 50° und 110°, vorzugsweise in einem Bereich zwischen 65° und 95°, insbesondere in einem Bereich zwischen 75° und 90° liegt.

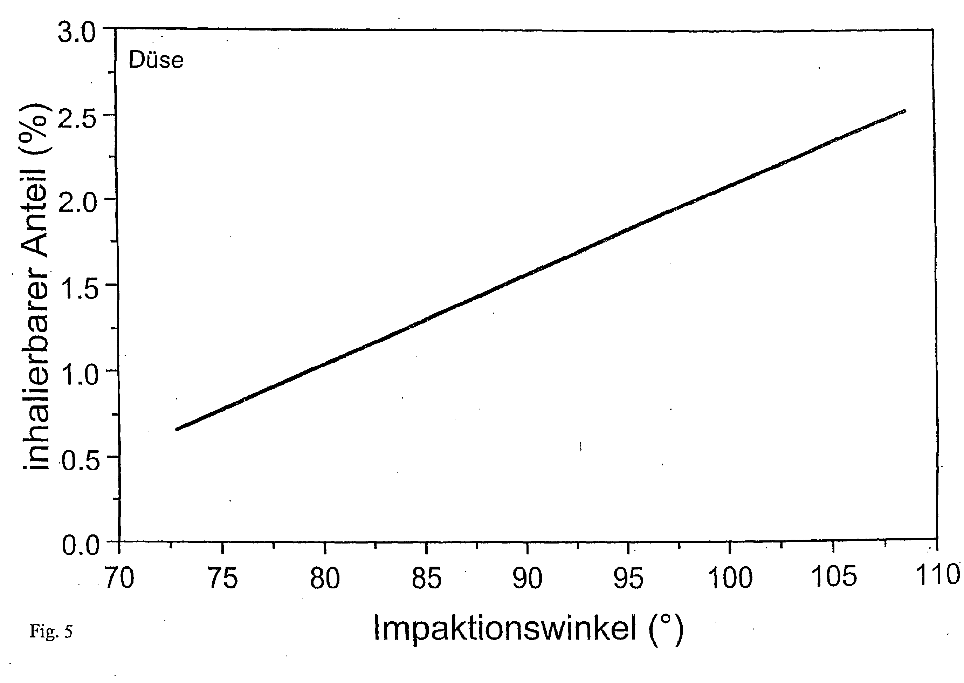

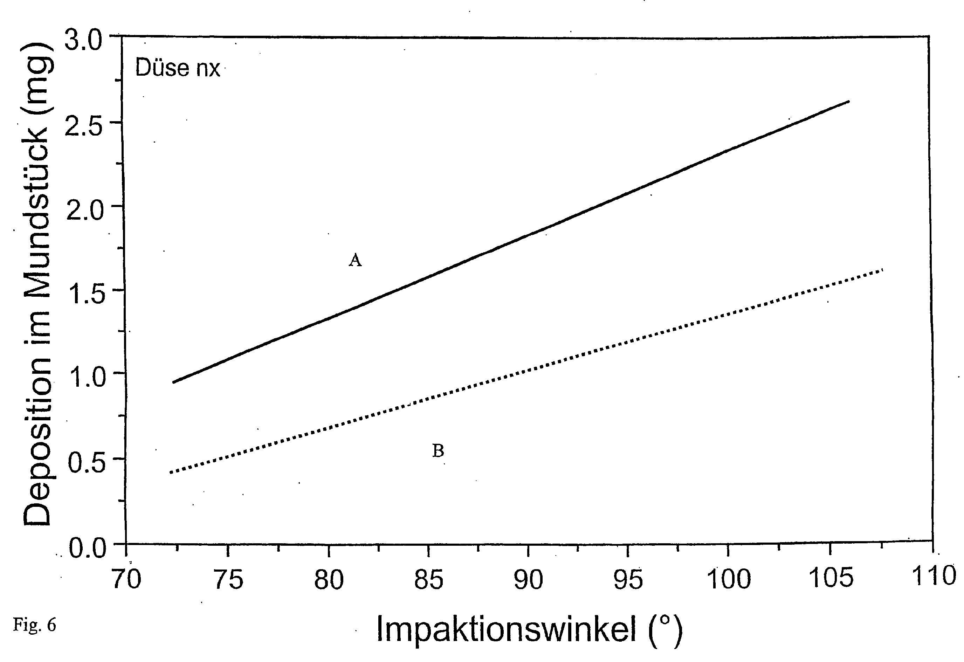

Die Figuren 5 und 6 zeigen den Einfluß des Impaktionswinkels α auf den inhalierbaren Anteil und den Mundstückteil. Beide wachsen mit zunehmendem Impaktionswinkel α. Im Hinblick auf die Zerstäubungsgüte ist ein möglichst frontales Zusammentreffen der Strahlen zu bevorzugen. Große Winkel sorgen für einen hohen inhalierbaren Anteil, d.h. für einen hohen Volumenanteil an kleinen Partikeln mit Durchmessern kleiner als 5,8µm in der Zerstäubungswolke.

Große Winkel α führen aber gleichzeitig zu großen Mundstückanteilen. Dabei darf der freie Weg, den die Strahlen zwischen dem Austritt aus den Düsenöffnungen und dem Auftreffpunkt zurücklegen, nicht zu groß gewählt werden, u.a. damit die Strahlen nicht vor dem Auftreffpunkt auseinanderfallen. Wird aber der Impaktionswinkel α vergrößert, muß die Impaktionshöhe verringert werden, um den freien Weg der Strahlen konstant zu halten. Die Auswirkungen dieser Maßnahme wurde bereits erläutert. Aber auch bei konstanter Impaktionshöhe und Vergrößerung des Winkels ergibt sich ein wachsender Mundstückanteil, da die Partikel der Zerstäubungswolke mit zunehmendem Impaktionswinkel einen wachsenden Impuls in Richtung Düsensystem erhalten, welcher letztendlich zu einem größeren Mundstückanteil führt. In den oben angegebenen Winkelbereichen läßt sich den konkurrierenden Mechanismen am besten Rechnung tragen.

Vorteilhaft sind Ausführungsformen der erfindungsgemäßen Düsen, bei denen der Abstand a der Düsenöffnungen in einem Bereich zwischen 40 µm und 125 µm, vorzugsweise in einem Bereich zwischen 50µm und 115 µm, insbesondere in einem Bereich zwischen 60 µm und 105 µm liegt.

Vorteilhaft sind Ausführungsformen des Düsensystems, welche dadurch gekennzeichnet sind, daß nur der Düsenhalter im montierten Zustand bis in den Bereich vor die Düsenöffnungen hinein reicht. Hierdurch werden Fugen zwischen Düsenhalter und Überwurfmutter im Bereich der Düsenöffnungen vermieden. Fugen sind hinsichtlich der Ablagerung von Aerosol-Partikeln besonders nachteilig, da hier einmal deponierte Partikel in der Regel nicht mehr freigesetzt werden.

Zwei Ausführungsformen gemäß der Zeichnungsfiguren 7, 8, 9 und 10 erläutern die Erfindung näher.

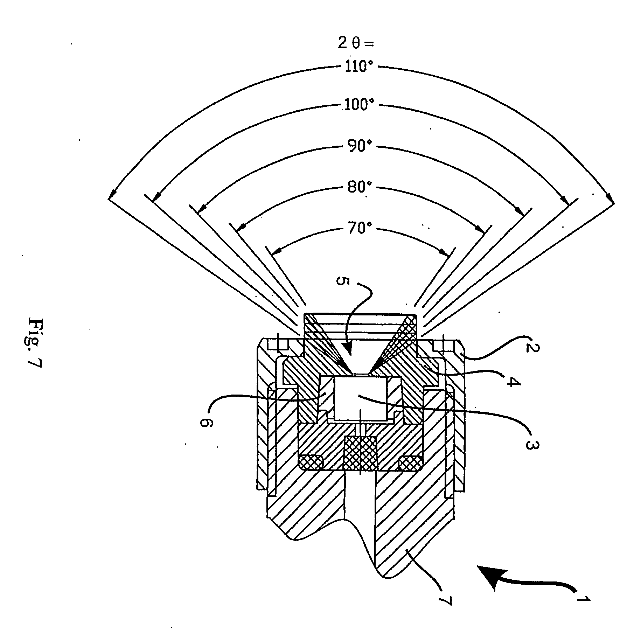

Figur 7 zeigt eine erste Ausführungsform des Düsensystems 1 in der Seitenansicht, teilweise geschnitten.

Die Düse 3 bzw. der Düsenkörper als selbständige bauliche Einheit - sogenannter Uniblock - ist in einer konischen Hülse 6 angeordnet, welche selbst wiederum im Düsenhalter 4 plaziert ist. Der Düsenhalter 4 wird mittels einer Überwurfmutter 2 am Gehäuse 7 verspannt und mit ihm wird die Düse 3 fixiert.

Dabei greift die Überwurfmutter 2 von außen in den Düsenhalter 4, wobei sie nicht bis in den Bereich vor die Düsenöffnungen hinein reicht. Die Ausnehmung 5 ist von konischer Gestalt in der Art, daß sie sich mit zunehmendem Abstand von den Düsenöffnungen kontinuierlich erweitert. Die Ausnehmung 5 weist einen Konuswinkel 2θ auf, wobei in Figur 7 beispielhaft mehrere, verschiedene Konuswinkel eingezeichnet sind, so daß diese Figur im Grunde gleich fünf verschiedene Ausführungen der Ausnehmung 5 und damit des Düsensystems 1 veranschaulicht. Im einzelnen sind Konuswinkel 2θ von 70°, 80°, 90°, 100° und 110° dargestellt.

Dadurch, daß die Überwurfmutter 2 von außen in den Düsenhalter 4 eingreift, wird die Ausnehmung 5 ausschließlich vom Düsenhalter 4 gebildet.

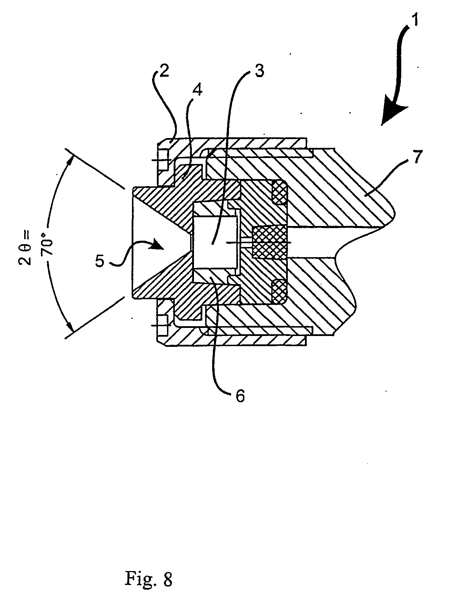

Im Gegensatz hierzu zeigt Figur 8 eine zweite Ausführungsform, ebenfalls in der Seitenansicht teilweise geschnitten, bei welcher sowohl der Düsenhalter 4 als auch die Überwurfmutter 2 bis in den Bereich vor die Düsenöffnungen hinein reichen. Im übrigen entspricht das in Figur 8 dargestellte Düsensystem 1 dem zuvor beschriebenen Düsensystem. Für gleiche Bauteile wurden dieselben Bezugszeichen verwendet, weshalb hinsichtlich der gleichartig ausgebildeten Bauteile auf die Beschreibung der Figur 7 verwiesen wird.

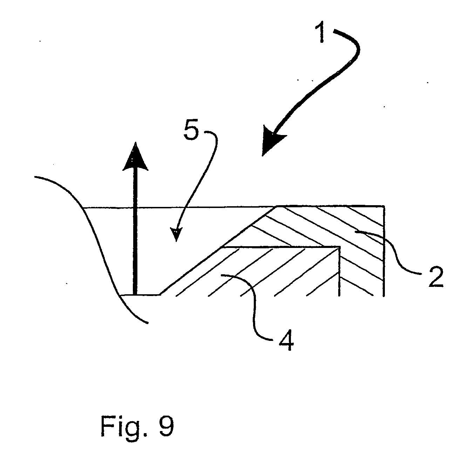

Figur 9 veranschaulicht noch einmal ein erfindungsgemäßes Düsensystem 1. Dieses weist eine Ausnehmung 5 von konischer Gestalt auf. Gegenüber Düsensystemen mit dis-kontinuierlich ausdehnender Ausnehmung findet sich in der Ausnehmung 5 keinerlei Stufe. Solche Stufen können insbesondere in diesem Bereich, auftreten, in dem die Überwurfmutter in den Düsenhalter eingreift. In diesen Fällen können sich Partikel der Zerstäuberwolke, an der Kante der Stufe ablagern und so zum Mundstückanteil beitragen.

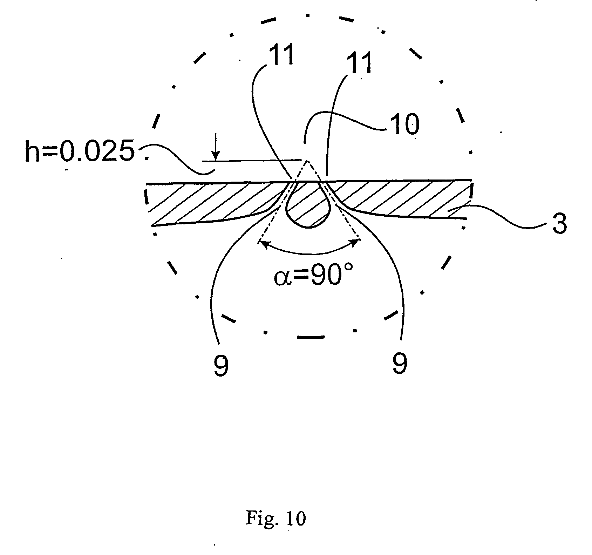

Figur 10 zeigt in einer schematischen Darstellung einen Ausschnitt einer Ausführungsform eines Düsenkörpers 3 in der Seitenansicht geschnitten.

Die beiden Düsenkanäle 9 sind in der Art angeordnet, daß die aus den Düsenöffnungen 11 der Düsenkanäle austretenden Strahlen im Kollisionspunkt 10 unter einem Winkel α=90° aufeinander treffen. Der Kollisionspunkt 10 hat eine Impaktionshöhe h=25µm über den Düsenöffnungen.

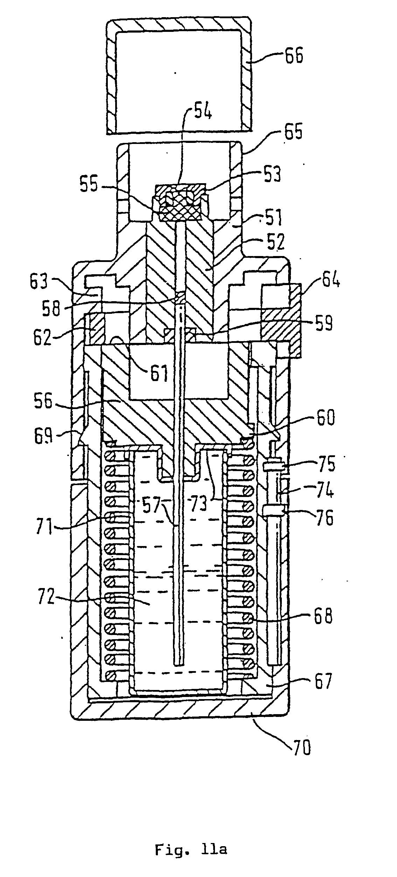

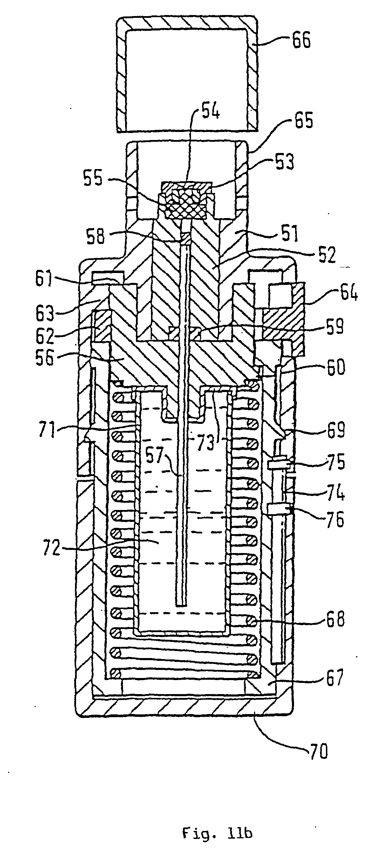

Figur 11 a zeigt einen Längsschnitt durch den Zerstäuber bei gespannter Feder. Figur 11b zeigt einen Längsschnitt durch den Zerstäuber bei entspannter Feder.
Das Gehäuseoberteil (51) enthält das Pumpengehäuse (52), an dessen Ende der Halter (53) für die Zerstäuberdüse angebracht ist. In dem Halter befindet sich die sich erweiternde Ausnehmung (54) und der Düsenkörper (55). Der im Abtriebsflansch (56) des Sperrspannwerkes befestigte Hohlkolben (57) ragt teilweise in den Zylinder des Pumpengehäuses hinein. An seinem Ende trägt der Hohlkolben den Ventilkörper (58). Der Hohlkolben ist mittels der Dichtung (59) abgedichtet. Innerhalb des Gehäuseoberteils befindet sich der Anschlag (60), an dem der Abtriebsflansch bei entspannter Feder anliegt. Am Abtriebsflansch befindet sich der Anschlag (61), an dem der Abtriebsflansch bei gespannter Feder anliegt. Nach dem Spannen der Feder schiebt sich das Sperrglied (62) zwischen den Anschlag (61) und eine Abstützung (63) im Gehäuseoberteil. Die Auslösetaste (64) steht mit dem Sperrglied in Verbindung. Das Gehäuseoberteil endet im Mundstück (65), welches mit der aufsteckbaren Schutzkappe (66) verschlossen ist.

Das Federgehäuse (67) mit Druckfeder (68) ist mittels der Schnappnasen (69) und Drehlager am Gehäuseoberteil drehbar gelagert. Über das Federgehäuse ist das Gehäuseunterteil (70) geschoben. Innerhalb des Federgehäuses befindet sich der austauschbare Vorratsbehälter (71) für das zu zerstäubende Fluid (72). Der Vorratsbehälter ist mit dem Stopfen (73) verschlossen, durch den der Hohlkolben in den Vorratsbehälter hineinragt und mit seinem Ende in das Fluid (Vorrat an Wirkstofflösung) eintaucht.

In der Mantelfläche des Federgehäuses ist die Spindel (74) für das mechanische Zählwerk angebracht (optional). An dem Ende der Spindel, das dem Gehäuseoberteil zugewandt ist, befindet sich das Antriebsritzel (75). Auf der Spindel sitzt der Reiter (76).

## Patentansprüche

1. Düsensystem (1), welches aus einer Düse (3) mit wenigstens zwei Düsenöffnungen (11) und einem Düsenhalter (4) und gegebenenfalls einer Überwurfinutter (2) besteht, wobei
- die Düsenöffnungen (11) bzw. in die Düsenöffnungen (11) mündenden Düsenkanäle (9) in der Art angeordnet sind, daß die aus den Düsenöffnungen (11) heraustretenden Strahlen in einem Winkel α aufeinandergelenkt werden,
- die Düse (3) im Düsenhalter (4) angeordnet ist und dieser gegebenenfalls von der Überwurfinutter (2) fixiert wird,
- im montierten Zustand der Düsenhalter (4) oder die Überwurfmutter (2) oder beide zumindest teilweise bis in den Bereich vor die Düsenöffnungen (11) hinein reichen,
- im montierten Zustand der Düsenhalter (4), oder wenn das Düsensystem (1) die Überwurfmutter (2) aufweist, der Düsenhalter (4) zusammen mit der Überwurfinutter (2) eine innere Ausnehmung aufweist,
- die an der an die Stirnseite der Düse (3) grenzenden Seite beginnt und sich bis zu der dazu parallel liegenden Außenseite des Düsenhalters (4) oder im Fall der Überwurfmutter (2) bis zu deren parallel zur Düsenstirnseite liegenden Außenseite erstreckt,
**dadurch gekennzeichnet daß**
- die Ausnehmung sich von der Stirnseite der Düse (3) aus betrachtet in Richtung der dazu parallel liegenden Außenseite des Düsenhalters (4) oder im Fall des Überwurfmutter (2) deren dazu parallel liegenden Außenseite stetig und kontinuierlich erweitert.

2. Düsensystem (1) nach Anspruch 1, **dadurch gekennzeichnet, daß** der Übergang zwischen Düsenhalter (4) und Uberwurfmutter (2) kantenlos ausgebildet ist

3. Düsensystem (1) nach einem der vorherigen Ansprüche, **dadurch gekennzeichnet, daß** die Ausnehmung (5) von trichterförmiger oder konusförmiger Gestalt ist.

4. Düsensystem (1) nach Anspruch 3, **dadurch gekennzeichnet, daß** die Ausnehmung (5) von konusförmiger Gestalt einen Konuswinkel 2θ aufweist, der im Bereich zwischen 55° und 155° liegt.

5. Düsensystem (1) nach Anspruch 3 oder 4, **dadurch gekennzeichnet, daß** die Ausnehmung (5) von konusförmiger Gestalt einen Konuswinkel 2θ aufweist, der im Bereich zwischen 70° und 140° liegt.

6. Düsensystem (1) nach einem der Ansprüche 3 bis 5, **dadurch gekennzeichnet, daß** die Ausnehmung (5) von konusförmiger Gestalt einen Konuswinkel 2θ aufweist, der im Bereich zwischen 70° bis 85° liegt.

7. Düsensystem (1) nach einem der Ansprüche 3 bis 5, **dadurch gekennzeichnet, daß** die Ausnehmung (5) von konusförmiger Gestalt einen Konuswinkel 20 aufweist, der im Bereich zwischen 95° und 140° liegt.

8. Düsensystem (1) nach einem der Ansprüche 3 bis 5 oder 7, **dadurch gekennzeichnet, daß** die Ausnehmung (5) von konusförmiger Gestalt einen Konuswinkel 2θ aufweist, der im Bereich zwischen 105° und 125° liegt.

9. Düsensystem (1) nach einem der vorherigen Ansprüche, **dadurch gekennzeichnet, daß** der Kollisionspunkt (10), in dem sich die Strahlen treffen, eine Impaktionshöhe h über den Düsenöffnungen (11) aufweist, die im Bereich zwischen 20µm und 85µm liegt.

10. Düsensystem (1) nach Anspruch 9, **dadurch gekennzeichnet, daß** der Kollisionspunkt (10), in dem sich die Strahlen treffen, eine Impaktionshöhe h über den Düsenöffnungen (11) aufweist, die im Bereich zwischen 25µm und 75µm liegt.

11. Düsensystem (1) nach Anspruch 9 oder 10, **dadurch gekennzeichnet, daß** der Kollisionspunkt (10), in dem sich die Strahlen treffen, eine Impaktionshöhe h über den Düsenöffnungen (11) aufweist, die im Bereich zwischen 35µm und 75µm liegt.

12. Düsensystem (1) nach einem der vorherigen Ansprüche, **dadurch gekennzeichnet, daß** der Winkel α in einem Bereich zwischen 50° und 110° liegt.

13. Düsensystem (1) nach Anspruch 12, **dadurch gekennzeichnet, daß** der Winkel α in einem Bereich zwischen 65° und 95° liegt.

14. Düsensystem (1) nach Anspruch 12 oder 13, **dadurch gekennzeichnet, daß** der Winkel α in einem Bereich zwischen 75° und 90° liegt.

15. Düsensystem (1) nach einem der vorherigen Ansprüche, **dadurch gekennzeichnet, daß** der Abstand a der Düsenöffnungen (11) in einem Bereich zwischen 40µm und 125 µm liegt.

16. Düsensystem (1) nach Anspruch 15, **dadurch gekennzeichnet, daß** der Abstand a der Düsenöffnungen (11) in einem Bereich zwischen 50µm und 115µm liegt.

17. Düsensystem (1) nach Anspruch 15 oder 16, **dadurch gekennzeichnet, daß** der Abstand a der Düsenöffnungen (11) in einem Bereich zwischen 60µm und 105µm liegt.

18. Düsensystem nach einem der vorangegangenen Ansprüche, **dadurch gekennzeichnet, dass** die Düse aus wenigstens zwei Baueinheiten gebildet wird.

19. Düsensystem nach einem der vorangegangenen Ansprüche, **dadurch gekennzeichnet, dass** die Düse aus wenigstens zwei aufeinanderliegenden Platten gebildet werden, wovon wenigstens eine der Platten eine zweite Mikrostruktur aufweist, so dass die übereinander liegenden Platten auf einer Seite einen Flüssigkeitseinlass definieren, dem sich ein Kanalsystem und/oder ein Filtersystem anschließt, welches dann in einen oder mehrere Flüssigkeitsauslässe mündet.

20. Ausbringungsvorrichtung für Flüssigkeiten, **dadurch gekennzeichnet, dass** sie ein Düsensystem nach einem der Ansprüche 1 bis 19 aufweist.

21. Ausbringungsvorrichtung nach Ansprüch 20, **dadurch gekennzeichnet, dass** es sich um einen Zerstäuber für pharmazeutische Flüssigkeiten handelt.

22. Ausbringungsvorrichtung nach einem der Ansprüche 20 oder 21, **dadurch gekennzeichnet, dass** die Vorrichtung ein unteres und ein oberes gegeneinander drehbar gelagertes Gehäuseteil besitzt, wobei in dem Gehäuseoberteil ein Federgehäuse mit Springfeder ausgebildet ist, welche durch Drehen der beiden Gehäuseteile über ein Sperrspannwerk bevorzugt in Form eines Schraubgewindes oder Getriebes gespannt und durch Drücken eines Auslöseknopfs am Gehäuseoberteil entspannt wird, wobei die Springfeder einen Abtriebsflansch bewegt, der mit einem Hohlkolben verbunden ist, an dessen unterem Ende ein Behälter aufgesteckt werden kann und an dessen oberem Ende sich ein Ventil und eine Druckkammer befindet, die mit der Düse oder dem Düsensystem, die (das) im nach oben hin offenen Bereich des Gehäuseoberteils ausgebildet ist, in einer eine Flüssigkeit leitenden Verbindung steht.

23. Ausbringungsvorrichtung nach einem der Ansprüche 20 bis 22, **dadurch gekennzeichnet, dass** es sich bei der Vorrichtung um einen Inhalator oder einen anderen Zerstäuber für medizinische Flüssigkeiten handelt.

## Claims

1. Nozzle system (1) which consists of a nozzle (3) having at least two nozzle apertures (11) and a nozzle holder (4) and optionally a check nut (2), wherein
- the nozzle apertures (11) or nozzle channels (9) opening into the nozzle apertures (11) are arranged so that the jets leaving the nozzle apertures (11) are aimed towards one another at an angle α,
- the nozzle (3) is arranged in the nozzle holder (4) and this is optionally fixed by the check nut (2),
- in the assembled state the nozzle holder (4) or the check nut (2) or both extend at least partially into the area in front of the nozzle apertures (11),
- in the assembled state the nozzle holder (4) or, if the nozzle system (1) has the check nut (2), the nozzle holder (4) together with the check nut (2) has an inner recess,
- which begins on the side adjacent to the end face of the nozzle (3) and extends as far as the outside of the nozzle holder (4) parallel thereto or, in the case of the check nut (2), as far as the outside thereof parallel to the end face of the nozzle,
**characterised in that** the recess, viewed from the end face of the nozzle (3), widens out steadily and continuously in the direction of the outside of the nozzle holder (4) parallel thereto or, in the case of the check nut (2), the outside of said check nut parallel thereto.

2. Nozzle system (1) according to claim 1, **characterised in that** the transition between nozzle holder (4) and check nut (2) is formed without any edges.

3. Nozzle system (1) according to one of the preceding claims, **characterised in that** the recess (5) is funnel-shaped or conical.

4. Nozzle system (1) according to claim 3, **characterised in that** the recess (5) of conical construction has a cone angle 2θ in the range between 55° and 155°.

5. Nozzle system (1) according to claim 3 or 4, **characterised in that** the recess (5) of conical construction has a cone angle 2θ in the range between 70° and 140°.

6. Nozzle system (1) according to one of claims 3 to 5, **characterised in that** the recess (5) of conical construction has a cone angle 2θ in the range between 70° and 85°.

7. Nozzle system (1) according to one of claims 3 to 5, **characterised in that** the recess (5) of conical construction has a cone angle 2θ in the range between 95° and 140°.

8. Nozzle system (1) according to one of claims 3 to 5 or 7, **characterised in that** the recess (5) of conical construction has a cone angle 20 in the range between 105° and 125°.

9. Nozzle system (1) according to one of the preceding claims, **characterised in that** the point of collision (10) where the jets meet has a height of impact h above the nozzle apertures (11) which is in the range between 20 µm and 85 µm.

10. Nozzle system (1) according to claim 9, **characterised in that** the point of collision (10) where the jets meet has a height of impact h above the nozzle apertures (11) which is in the range between 25 µm and 75 µm.

11. Nozzle system (1) according to claim 9 or 10, **characterised in that** the point of collision (10) where the jets meet has a height of impact h above the nozzle apertures (11) which is in the range between 35 µm and 75 µm.

12. Nozzle system (1) according to one of the preceding claims, **characterised in that** the angle α is in the range between 50° and 110°.

13. Nozzle system (1) according to claim 12, **characterised in that** the angle α is in the range between 65° and 95°.

14. Nozzle system (1) according to claim 12 or 13, **characterised in that** the angle α is in the range between 75° and 90°.

15. Nozzle system (1) according to one of the preceding claims, **characterised in that** the spacing a of the nozzle apertures (11) is in the range between 40 µm and 125 µm.

16. Nozzle system (1) according to claim 15, **characterised in that** the spacing a of the nozzle apertures (11) is in the range between 50 µm and 115 µm.

17. Nozzle system (1) according to claim 15 or 16, **characterised in that** the spacing a of the nozzle apertures (11) is in the range between 60 µm and 105 µm.

18. Nozzle system according to one of the preceding claims, **characterised in that** the nozzle is formed from at least two construction units.

19. Nozzle system according to one of the preceding claims, **characterised in that** the nozzle is formed from at least two superimposed plates, at least one of the plates having a second microstructure so that the plates lying one on top of the other define, on one side, a liquid inlet connected to a channel system and/or a filter system which then opens into one or more liquid outlets.

20. Delivery device for liquids, **characterised in that** it comprises a nozzle system according to one of claims 1 to 19.

21. Delivery device according to claim 20, **characterised in that** it is an atomiser for pharmaceutical liquids.

22. Delivery device according to one of claims 20 or 21, **characterised in that** the device comprises a lower and an upper housing part mounted to be rotatable relative to one another, the upper part of the housing containing a spring housing with spring which is tensioned by rotating the two housing parts by means of a locking clamping mechanism preferably in the form of a screw thread or gear and is released by pressing a release button on the upper part of the housing, the spring meanwhile moving a power take-off flange connected to a hollow piston on the lower end of which a container can be fitted and at the upper end of which are found a valve and a pressure chamber which is connected for fluid transmission to the nozzle or the nozzle system formed in the upwardly open part of the upper housing part.

23. Delivery device according to one of claims 20 to 22, **characterised in that** the device is an inhaler or some other atomiser for medicinal liquids.

## Revendications

1. Système de buse (1) qui est constitué d'une buse (3) comportant au moins deux ouvertures de buse (11) et d'un support de buse (4) et éventuellement d'un écrou-raccord (2), dans lequel
- les ouvertures de buse (11) respectivement des canaux de buse (9) débouchant dans les ouvertures de buse (11) sont disposés de telle sorte que les jets sortants des ouvertures de buse (11) soient dirigés l'un sur l'autre selon un angle α,
- la buse (3) est disposée dans le support de buse (4) et celui-ci est fixé éventuellement par l'écrou-raccord (2),
- à l'état monté, le support de buse (4) ou l'écrou-raccord (2) ou les deux atteignent au moins partiellement la zone se trouvant devant les ouvertures de buse (11),
- à l'état monté, le support de buse (4) ou, lorsque le système de buse (1) présente l'écrou-raccord (2), le support de buse (4) conjointement avec l'écrou-raccord (2) présentent un évidement interne,
- qui commence du côté adjacent au côté frontal de la buse (3) et qui s'étend jusqu'au côté externe du support de buse (4) se trouvant en parallèle par rapport celui-ci ou, dans le cas de l'écrou-support (2), jusqu'à son côté externe se trouvant en parallèle par rapport au côté frontal de la buse,
**caractérisé en ce que**
- l'évidement s'élargit constamment et en continu du côté frontal de la buse (3), considéré dans la direction du côté extérieur du support de buse (4) se trouvant en parallèle de celui-ci ou, dans le cas de l'écrou-raccord (2), de son côté extérieur se trouvant en parallèle.

2. Système de buse (1) selon la revendication 1, **caractérisé en ce que** la transition entre le support de buse (4) et l'écrou-raccord (2) est constitué sans arête.

3. Système de buse (1) selon l'une des revendications précédentes, **caractérisé en ce que** l'évidement (5) est en forme d'entonnoir ou de cône.

4. Système de buse (1) selon la revendication 3, **caractérisé en ce que** l'évidement (5) de configuration conique présente un angle de cône 2θ qui se situe dans une plage comprise entre 55° et 155°.

5. Système de buse (1) selon la revendication 3 ou 4, **caractérisé en ce que** l'évidement (5) de forme conique présente un angle de cône 2θ qui se situe dans une plage comprise entre 70° et 140°.

6. Système de buse (1) selon l'une des revendications 3 à 5, **caractérisé en ce que** l'évidement (5) de forme conique présente un angle de cône 2θ qui se situe dans une plage comprise entre 70° et 85°.

7. Système de buse (1) selon l'une des revendications 3 à 5, **caractérisé en ce que** l'évidement (5) de forme conique présente un angle de cône 2θ qui se situe dans une plage comprise entre 95° et 140°.

8. Système de buse (1) selon l'une des revendications 3 à 5 ou 7, **caractérisé en ce que** l'évidement (5) de forme conique présente un angle de cône 2θ qui se situe dans une plage comprise entre 105° et 125°.

9. Système de buse (1) selon l'une des revendications précédentes, **caractérisé en ce que** le point d'impact (10) au niveau duquel les jets se rencontrent présente une hauteur d'impact h au-dessus des ouvertures de buse (11) qui se situe dans une plage comprise entre 20 µm et 85 µm.

10. Système de buse (1) selon la revendication 9, **caractérisé en ce que** le point d'impact (10) au niveau duquel les jets se rencontrent présente une hauteur d'impact h au-dessus des ouvertures de buse (11) qui se situe dans une plage comprise entre 25 µm et 75 µm.

11. Système de buse (1) selon la revendication 9 ou 10, **caractérisé en ce que** le point d'impact (10) au niveau duquel les jets se rencontrent présente une hauteur d'impact h au-dessus des ouvertures de buse (11) qui se situe dans une plage comprise entre 35 µm et 75 µm.

12. Système de buse (1) selon l'une des revendications précédentes, **caractérisé en ce que** l'angle α se situe dans une plage comprise entre 50° et 110°.

13. Système de buse (1) selon la revendication 12, **caractérisé en ce que** l'angle α se situe dans une plage comprise entre 65° et 95°.

14. Système de buse (1) selon la revendication 12 ou 13, **caractérisé en ce que** l'angle α se situe dans une plage comprise entre 75° et 90°.

15. Système de buse (1) selon l'une des revendications précédentes, **caractérisé en ce que** l'intervalle "a" des ouvertures de buse (11) se situe dans une plage comprise entre 40 µm et 125 µm.

16. Système de buse (1) selon la revendication 15, **caractérisé en ce que** l'intervalle "a" des ouvertures de buse (11) se situe dans une plage comprise entre 50 µm et 115 µm.

17. Système de buse (1) selon la revendication 15 ou 16, **caractérisé en ce que** l'intervalle "a" des ouvertures de buse (11) se situe dans une plage comprise entre 60 µm et 105 µm.

18. Système de buse selon l'une des revendications précédentes, **caractérisé en ce que** la buse est constituée d'au moins deux unités.

19. Système de buse selon l'une des revendications précédentes, **caractérisé en ce que** la buse est formée d'au moins deux plaques superposées, au moins l'une des plaques présentant une deuxième microstructure de telle sorte que les plaques superposées définissent d'un côté une entrée de liquide à laquelle se raccorde un système de canal et/ou un système de filtre qui débouche ensuite dans une ou plusieurs sorties de liquide.

20. Dispositif de distribution de liquides, **caractérisé en ce qu'**il présente un système de buse selon l'une des revendications 1 à 19.

21. Dispositif de distribution selon la revendication 20, **caractérisé en ce qu'**il s'agit d'un pulvérisateur de liquides pharmaceutiques.

22. Dispositif de distribution selon l'une des revendications 20 ou 21, **caractérisé en ce que** le dispositif possède une partie de logement inférieure et une partie de logement supérieure placée de façon à pouvoir tourner l'un contre l'autre, un logement de ressort doté d'un ressort de pression étant constitué dans la partie de logement supérieure, lequel ressort est tendu en tournant les deux parties de logement sur un tendeur bloqué, de préférence sous la forme d'un raccord à vis ou d'un engrenage, et est détendu en appuyant sur un bouton de détente situé sur la partie de logement supérieure, le ressort de pression déplaçant une bride d'entraînement qui est reliée à un piston creux, au niveau de l'extrémité inférieure duquel un récipient peut être attaché et, au niveau de l'extrémité supérieure duquel se trouvent une soupape et une chambre de pression qui est en communication de fluide avec la buse ou le système de buse qui est constitué(e) dans la zone ouverte vers le haut de la partie supérieure du logement.

23. Dispositif de distribution selon l'une des revendications 20 à 22, **caractérisé en ce que** le dispositif est un inhalateur ou un pulvérisateur de liquides médicaux.
